# EUROPEAN PATENT APPLICATION

(11) **EP 4 550 346 A1**
(43) Date of publication of application: **07.05.2025**
(21) Application number: 23207971.5
(22) Date of filing: 06.11.2023
(51) Int. Cl.: G16H 30/00, G16H 40/67

(54) **TECHNIQUE FOR PROCESSING AND LOCALLY OUTPUTTING AUDIO INPUT RECEIVED FROM A REMOTE OPERATOR IN A MEDICAL SYSTEM**

(71) Applicant: Roclub GmbH, 10627 Berlin (DE)
(72) Inventor: ISSING, Matthias, 10627 Berlin (DE)
(74) Representative: Laqua, Bernd Christian Kurt

(57) **Abstract**

A system (100) for processing and locally outputting audio input received from a remote operator in a medical system supporting medical remote operation and a method for operating the system (100) are presented. The system (100) comprises a local computing device (200). The local computing device (200) comprises a first audio interface (210) configured for processing audio input, wherein a first audio device (400) is connected to the first audio interface (210) and wherein the first audio device (400) is configured to output the audio input processed by the first audio interface (210) to a local operator of a medical imaging device (500), and a second audio interface (220) configured for processing audio input, wherein a second audio device (510) is connected to the second audio interface (220) and wherein the second audio device (510) is configured to output the audio input processed by the second audio interface (220) to a patient situated in the medical imaging device (500). The local computing device (200) further comprises a processor (230) configured to receive audio input from the remote operator and control input from the remote operator, select, based on the control input received from the remote operator, one of the first audio interface (210) and the second audio interface (220) for processing the audio input received from the remote operator, output the audio input processed by the first audio interface (210) via the first audio device (400) when the first audio interface (210) is selected based on the received control input, and output the audio input processed by the second audio interface (220) via the second audio device (510) when the second audio interface (220) is selected based on the received control input.

## Description

### Technical Field

The present disclosure generally relates to medical systems. In particular, a system for processing and locally outputting audio input received from a remote operator in a medical system supporting medical remote operation and a method for operating the system are presented. Furthermore, a computer program product is presented.

### Background

Medical imaging techniques like Magnetic Resonance Imaging (MRI) or Computed Tomography (CT) have a variety of applications in modern medicine. Performing corresponding medical imaging examinations is generally very complex and requires a person with special training, i.e., a radiographer, to be present at the site where the examination is performed. The radiographer has the task of adjusting a medical imaging device via a workstation in order to examine the desired body region and to achieve the best possible image quality. For examining specific questions, specialized knowledge and extensive experience are required.

However, there is a severe shortage of these professionals worldwide, making it challenging for operators of medical imaging devices, e.g., hospitals or medical imaging centres, to have an adequately qualified radiographer available on site at the right time.

To solve this problem, various medical systems exist that support medical remote operation. For example, the workstation of the medical imaging device may be remotely operable enabling the radiographer to work as a remote operator of the medical imaging device. Thus, the radiographer no longer needs to be on site for an examination. Instead, it is sufficient that a local operator with lower qualifications is on site to take over the remaining tasks that have to be performed on site, e.g., positioning and care of a patient situated in the medical imaging device.

However, during an MRI or CT examination, it may be necessary for the radiographer to give the patient voice instructions, e.g., breathing commands or other instructions. Commercially available medical imaging devices commonly offer a local speech device (e.g., InterCom), enabling the local operator to communicate with the patient situated in the medical imaging device. For example, the local operator presses and holds a button of the speech device to activate a microphone thereof. The instructions of the local operator may then be output to the patient via a special headset or a speaker.

To further enable the remote operator to give instructions to the patient, common techniques may provide a mechanical actuator, e.g., a robotic arm controllable by the remote operator for pressing and holding the button of the speech device. The instructions from the remote operator may be output via a speaker to the microphone of the speech device and then transmitted by the speech device to the patient.

However, such a technique is technically complex and error-prone. It can lead to malfunctions of the mechanical actuator, slippage of the position and losses in speech quality, for example.

### Summary

There is a need for a technique for allowing a remote operator of a medical system supporting medical remote operation to reliably provide instructions to a local operator of the medical imaging device and to a patient situated in the medical imaging device.

According to a first aspect, a system for processing and locally outputting audio input received from a remote operator in a medical system supporting medical remote operation is provided. The system comprises a local computing device. The local computing device comprises a first audio interface configured for processing audio input, wherein a first audio device is connected to the first audio interface and wherein the first audio device is configured to output the audio input processed by the first audio interface to a local operator of a medical imaging device, and a second audio interface configured for processing audio input, wherein a second audio device is connected to the second audio interface and wherein the second audio device is configured to output the audio input processed by the second audio interface to a patient situated in the medical imaging device. The local computing device further comprises a processor configured to receive audio input from the remote operator and control input from the remote operator, select, based on the control input received from the remote operator, one of the first audio interface and the second audio interface for processing the audio input received from the remote operator, output the audio input processed by the first audio interface via the first audio device when the first audio interface is selected based on the received control input, and output the audio input processed by the second audio interface via the second audio device when the second audio interface is selected based on the received control input.

The local computing device may be or comprise a commercially available computing device like a desktop computer, a laptop, or a workstation. The local computing device may be located in a room next to an examination room in which the medical imaging device is located. The local computing device may be connected to a workstation of the medical imaging device, e.g., for at least one of operating the medical imaging device and exchanging data therewith. In particular, one or more settings and/or functions of the medical imaging device may be controllable via the local computing device. The local computing device may be connected to the workstation of the medical imaging device wirelessly or by a wired connection.

The local computing device may further be configured to receive data from a remote computing device operated by the remote operator. For example, the local computing device may host a software application accessible through the remote computing device. The software application may be configured to enable at least one of operation of the medical imaging device via the remote computing device, receiving data from the remote computing device and transmitting data to the remote computing device. The received data may comprise at least one of the audio input from the remote operator and the control input from the remote operator, wherein the audio input may comprise voice instructions from the remote operator. The remote computing device may be or comprise a commercially available computing device like a desktop computer, a laptop or a workstation, for example.

The first audio interface and the second audio interface may be a first sound card and a second sound card, respectively. For example, the sound cards may be common, commercially available sound cards. In one variant, the first and second sound cards may be identical types of sound cards. The first audio interface may be connected to the first audio device wirelessly or by a wired connection. The second audio interface may be connected to the second audio device wirelessly or by a wired connection.

The first audio device may be or comprise a speaker, e.g., a commercially available speaker. The second audio device may be or comprise a speaker, e.g., a commercially available speaker. The second audio device may be integrated into the medical imaging device. For example, the second audio device may be or may be part of a local speech device of the medical imaging device, such as an InterCom, and the second audio interface may be connected to an input of the local speech device.

The medical imaging device may be any known image device, for example, a CT or an MRI scanner. The remote operator may be a radiographer and the local operator may be medical personnel present at the site, where the medical imaging device resides.

In some variants, the processor of the local computing device may be configured to select the first audio interface per default when no control input from the remote operator is received and to select the second audio interface when the control input is received from the remote operator. In other words, the selection may be a binary decision based on whether or not control input is received.

The control input may be received from the remote operator for a period of time and the selection may be changed from the second audio interface to the first audio interface after the period of time lapses.

Alternatively, the control input may indicate which of the first and second audio interfaces is to be selected. In this case, a currently selected audio interface may be changed upon receiving new control input indicating that an audio interface is to be used that is currently not selected.

In some variants, the first and second audio interfaces may be configurable to be assigned to either the first audio device or the second audio device and the processor of the local computing device may be further configured to receive configuration input from the local operator indicating that the first audio interface is to be assigned to the first audio device and that the second audio interface is to be assigned to the second audio device. The configuration input may enable a flexible and selective configuration of the system by the local operator, in particular when the audio interfaces are no identical interfaces and are wirelessly connectable to the audio devices, for example.

The audio input processed by the first audio interface for output to the local operator may be part of an audio or video call held between the local operator and the remote operator. The audio or video call may be carried out using a dedicated software application installed on the local computing device and on the remote computing device. Alternatively, the audio or video call may be a browser-based application, for example. The audio input processed by the second audio interface for output to the patient situated in the medical imaging device, on the other hand, may be for communication between the remote operator and the patient. In particular, the audio input processed by the second audio interface may comprise instructions from the remote operator to the patient, like breathing instructions, or any other kind of instructions to the patient.

The control input may be indicative of the remote operator actuating an input element. The input element may be a virtual input element of a software application (e.g., the dedicated software application installed on the local computing device, or the browser-based application). The input element may be a button. In particular, the button may be a virtual button shown in the software application on the remote computing device to the remote operator. The remote operator may actuate the button, e.g., via a computer mouse or touchscreen or any other input device. The control input may be received as long as the remote operator (permanently) actuates the button, for example. Alternatively, the control input may be received upon a first actuation of the button (e.g., indicating a start of the selection of the second audio interface) as well as upon a second actuation of the button (e.g., indicating an end of the selection of the second audio interface).

In some variants, the local computing device may further comprise a display and the processor of the local computing device may be further configured to trigger, via the display, an indication to the local operator indicative of the remote operator actuating the input element. The indication may correspond to the button shown to the remote operator, for example. Additionally or alternatively, the indication may comprise a popup event, for example. Still additionally or alternatively, the indication may comprise a change of colour and/or size of an element shown on the display.

According to a second aspect, a method for operating a system for processing and locally outputting audio input received from a remote operator in a medical system supporting medical remote operation is provided. The method is performed by a processor of a local computing device of the system and comprises receiving audio input from the remote operator and control input from the remote operator. The method further comprises selecting, based on the control input received from the remote operator and for processing the audio input received from the remote operator, one of i) a first audio interface of the local computing device, the first audio interface being configured for processing audio input, wherein a first audio device is connected to the first audio interface and wherein the first audio device is configured to output the audio input processed by the first audio interface to a local operator of a medical imaging device, and ii) a second audio interface of the local computing device, the second audio interface being configured for processing audio input, wherein a second audio device is connected to the second audio interface and wherein the second audio device is configured to output the audio input processed by the second audio interface to a patient situated in the medical imaging device. The method further comprises outputting the audio input processed by the first audio interface via the first audio device when the first audio interface is selected based on the received control input, and outputting the audio input processed by the second audio interface via the second audio device when the second audio interface is selected based on the received control input.

The system operated by the method of the second aspect may correspond to the system according to the first aspect. As such, those aspects described above regarding the system of the first aspect may likewise be reflected in the method of the second aspect in the form of corresponding method steps or characteristics. Unnecessary repetitions are thus omitted in the following.

As in the system of the first aspect, the selecting step may comprise selecting the first audio interface per default when no control input from the remote operator is received and selecting the second audio interface when the control input is received from the remote operator.

The control input may be received from the remote operator for a period of time and the method may further comprise changing the selection from the second audio interface to the first audio interface after the period of time lapses.

In some variants, the first and second audio interfaces may be configurable to be assigned to either the first audio device or the second audio device, and the method may further comprise receiving configuration input from the local operator indicating that the first audio interface is to be assigned to the first audio device and that the second audio interface is to be assigned to the second audio device.

In some variants of the method, the audio input processed by the first audio interface for output to the local operator may be part of an audio or video call held between the local operator and the remote operator, and the audio input processed by the second audio interface for output to the patient situated in the medical imaging device may be for communication between the remote operator and the patient.

The control input may be indicative of the remote operator actuating an input element. The input element may be a button.

In some variants, the local computing device may further comprise a display, and the method may further comprise triggering, via the display, an indication to the local operator indicative of the remote operator actuating the input element.

According to a third aspect, a computer program product is provided. The computer program product comprises instructions that, when executed on at least one processor, cause the at least one processor to carry out the method according to the second aspect or any variant thereof described herein. The computer program product may be stored on a computer readable recording medium, such as a semiconductor memory, DVD, CD-ROM, and so on. The computer program product may also be provided for download via a communication network (e.g., the Internet or a proprietary network).

Furthermore, it is noted that, when in the following as well as in relation to any of the aspects disclosed herein, it is said that the processor of the local computing device is "configured to" perform some action or method step, it will be understood by one of skill in the art that the processor is provided together with at least one memory, wherein the at least one memory contains instructions executable by the processor such that the processor is operable to carry out the respective action or method step described herein.

### Brief Description of the Drawings

Further details, advantages and aspects of the present disclosure will become apparent from the following embodiments taken in conjunction with the drawings, wherein:
- Fig. 1: shows a schematic representation of a system for processing and locally outputting audio input received from a remote operator in a medical system supporting medical remote operation;
- Fig. 2: shows a flow diagram of a method for operating the system of Fig. 1; and
- Fig. 3: shows a schematic representation of computer program product comprising instructions for executing the method.

### Detailed Description

In the following description, for purposes of explanation and not limitation, specific details are set forth in order to provide a thorough understanding of the present disclosure. It will be apparent to one skilled in the art that the present disclosure may be practiced in other embodiments that depart from these specific details. In the following description of exemplary embodiments, the same reference numerals are used to denote the same or similar components.

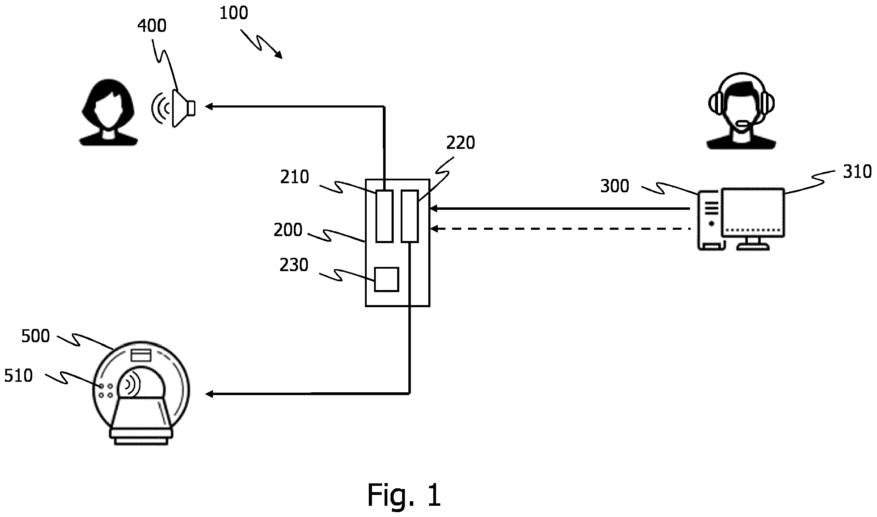

Fig. 1 shows a schematic representation of a system 100 for processing and locally outputting audio input received from a remote operator in a medical system supporting medical remote operation. In the variant shown in Fig. 1, the remote operator is a remotely located radiographer (referred to in the following as radiographer) and the system 100 enables the radiographer to selectively provide instructions to a local operator (e.g., medical personnel present at a site where a medical imaging device in which a patient is to be examined resides), or to a patient being examined (not shown).

The system 100 comprises a local computing device 200 (e.g., residing locally at the site where the medical imaging device is provided) with a first audio interface 210 configured for processing audio input and a second audio interface 220 configured for processing audio input. In the variant shown in Fig. 1, the local computing device 200 is a local desktop computer 200 and the first and second audio interfaces 210, 220 are sound cards 210, 220.

A processor 230 of the local desktop computer 200 is configured to receive input from the radiographer, e.g., via a remote computing device 300 the radiographer is operating. The remote computing device 300 may be located in a different location (e.g., building or city) distant from the site where the medical imaging device resides, so that medical remote operation is enabled for the remote operator (who is not on-site). The remote computing device 300 shown in Fig. 1 comprises a desktop computer 300 with a headset connected thereto. The headset has a microphone for generating audio input, e.g., data indicative of speech input from the radiographer. The remote desktop computer 300 is configured to transmit the audio input to the local desktop computer 200. In other variants, the radiographer may use different remote computing devices 300 to provide audio input to the local desktop computer 200. In particular, any computing device 300 configured to generate audio input from speech input from the radiographer and to transmit the audio input to the local desktop computer 200 may be utilized. In a particular variant, the radiographer and the local operator communicate through an audio or video call, wherein both the radiographer and the local operator are logged into the call via a browser-based or dedicated software application, for example.

In addition to the audio input, the radiographer can generate control input, e.g., via an input device like a computing mouse (not shown) connected to the remote desktop computer 300 or a touchscreen of a remote computing device 300. The control input may indicate which of the first and the second soundcard 210, 220 is to be selected for processing the audio input of the radiographer. Generating the control input may comprise actuating a virtual button shown on the display 310 of the remote desktop computer 300, for example.

In the shown variant, the processor 230 of the local desktop computer 200 may be configured to select the first sound card 210 by default for processing the audio input received from the radiographer, when no control input is received. This audio input may relate to the audio or video call between the local operator and the remote operator. Processing the received audio input via the sound card 210 may comprise using known techniques for improving the quality of the received audio input. The processor 230 of the local desktop computer 200 is further configured to output the audio input processed by the first sound card 210 via a first audio device 400 to the local operator. The first audio device 400 shown in Fig. 1 is a speaker 400 located at the site of the local operator so that the local operator can receive instructions from the radiographer via the audio input, e.g., as part of the audio or video call.

In case the radiographer wants to communicate with a patient situated in a medical imaging device 500 instead of the local operator, the radiographer may generate corresponding control input. The control input may be generated simultaneously with the audio input, for example. When the processor 230 of the local desktop computer 300 receives the control input together with the audio input (e.g., simultaneously), the processor 230 changes the selection from the first sound card 210 to the second sound card 220 for processing the audio input. The processor 230 of the local desktop computer 200 is further configured to output the audio input processed by the second sound card 220 via a second audio device 510 to the patient situated in the medical imaging device 500. The second audio device 510 shown in Fig. 1 is a speaker 510 of (e.g., integrated into) the medical imaging device 500. In other variants, the speaker 510 may be a stand-alone speaker located in the examination room where the medical imaging device 500 resides. Further implementations of a speaker are contemplated as long as the speaker enables outputting of the audio input processed by the second sound card 220 to the patient.

As a result, it may be summarized that the radiographer may select one of the sound cards 210, 220 for processing the audio input by generating corresponding control input, in order to thereby select the addressee for his instructions. Since the audio input from the radiographer is directly output at speakers 400, 510 located in the vicinity of the addressee, high quality of the audio is ensured for the desired recipient. Furthermore, by selectively switching between the first and second sound cards 210, 220 via actuation of an input element (not shown), such as a virtual button available in the audio or video call software, an easy to operate system 100 with minimal risk of malfunction is provided.

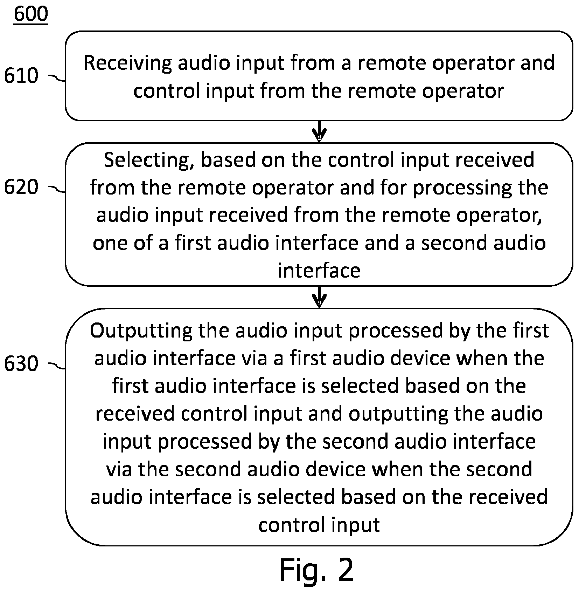

Fig. 2 shows a flow diagram of a method 600 for operating the system 100 of Fig. 1. The method 600 comprises a first step 610 of receiving audio input from the remote operator, e.g., the radiographer, and control input from the remote operator. The audio input and control input may be generated as described with reference to Fig. 1.

The method 600 further comprises a second step 620 of selecting, based on the control input received from the remote operator and for processing the audio input received from the remote operator, one of the first audio interface 210, e.g., the first sound card 210, and the second audio interface 220, e.g., the second sound card.

As explained with reference to Fig. 1, in one variant, the first audio interface 210 may be selected per default when no control input is received and the second audio interface 220 may be selected when control input is received. In one particular variant, the second audio interface 220 may be selected as long as control input is received and the selection may be automatically changed back to the first audio interface 210 when the control input is no longer received. In other variants, the control information may be indicative of the audio interface 210, 220 that is to be selected and a change of the selection may only be performed when new control input indicating such change is received.

A third step 630 of the method 600 comprises outputting the audio input processed by the first audio interface 210 via the first audio device 400, e.g., a speaker configured for outputting the processed audio input to the local operator, when the first audio interface 210 is selected based on the received control input, and outputting the audio input processed by the second audio interface 220 via the second audio device 510, e.g., a speaker configured for outputting the processed audio input to the patient situated in the medical imaging device 500, when the second audio interface 220 is selected based on the received control input.

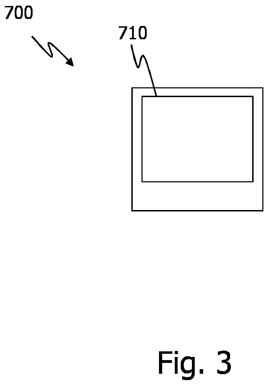

Fig. 3 shows a schematic representation of a computer program product 700 comprising instructions 710 configured to perform the steps of the method 700 described herein, when executed on one or more processor, e.g., on the one or more processors (not shown) of the local computing device 200 shown in Fig. 1.

As has become apparent from the above, the present disclosure provides a technique that enables reliably providing instructions from a remote operator, e.g., radiographer, selectively to either a local operator of a medical imaging device or a patient situated in the medical imaging device.

It is believed that the advantages of the technique presented herein will be fully understood from the foregoing description, and it will be apparent that various changes may be made in the form, constructions and arrangement of the exemplary aspects thereof without departing from the scope of the invention or without sacrificing all of its advantageous effects. Because the technique presented herein can be varied in many ways, it will be recognized that the invention should be limited only by the scope of the claims that follow.

## Claims

1. A system (100) for processing and locally outputting audio input received from a remote operator in a medical system supporting medical remote operation, wherein the system (100) comprises:
a local computing device (200) comprising:
a first audio interface (210) configured for processing audio input, wherein a first audio device (400) is connected to the first audio interface (210) and wherein the first audio device (400) is configured to output the audio input processed by the first audio interface (210) to a local operator of a medical imaging device (500);
a second audio interface (220) configured for processing audio input, wherein a second audio device (510) is connected to the second audio interface (220) and wherein the second audio device (510) is configured to output the audio input processed by the second audio interface (220) to a patient situated in the medical imaging device (500); and
a processor (230) configured to:
receive (610) audio input from the remote operator and control input from the remote operator;
select (620), based on the control input received from the remote operator, one of the first audio interface (210) and the second audio interface (220) for processing the audio input received from the remote operator;
output (630) the audio input processed by the first audio interface (210) via the first audio device (400) when the first audio interface (210) is selected based on the received control input; and
output (630) the audio input processed by the second audio interface (220) via the second audio device (510) when the second audio interface (220) is selected based on the received control input.

2. The system (100) of claim 1, wherein
the processor (230) of the local computing device (200) is further configured to select the first audio interface (210) per default when no control input from the remote operator is received and to select the second audio interface (220) when the control input is received from the remote operator.

3. The system (100) of claim 2, wherein
the control input is received from the remote operator for a period of time and wherein the selection is changed from the second audio interface (220) to the first audio interface (210) after the period of time lapses.

4. The system (100) of any preceding claim, wherein
the first and second audio interfaces (210, 220) are configurable to be assigned to either the first audio device (400) or the second audio device (510), wherein the processor (230) of the local computing device (200) is further configured to receive configuration input from the local operator indicating that the first audio interface (210) is to be assigned to the first audio device (400) and that the second audio interface (220) is to be assigned to the second audio device (510).

5. The system (100) of any preceding claim wherein
the audio input processed by the first audio interface (210) for output to the local operator is part of an audio/video call held between the local operator and the remote operator, and wherein the audio input processed by the second audio interface (220) for output to the patient situated in the medical imaging device (500) is for communication between the remote operator and the patient.

6. The system (100) of any preceding claim, wherein
the control input is indicative of the remote operator actuating an input element; and, optionally, wherein
the input element is a button.

7. The system (100) of claim 6, wherein
the local computing device (200) further comprises a display; and wherein
the processor (230) of the local computing device (200) is further configured to trigger, via the display, an indication to the local operator indicative of the remote operator actuating the input element.

8. A method (600) for operating a system (100) for processing and locally outputting audio input received from a remote operator in a medical system supporting medical remote operation, the method (600) being performed by a processor (230) of a local computing device (200) of the system (100) and comprising:
receiving (610) audio input from the remote operator and control input from the remote operator;
selecting (620), based on the control input received from the remote operator and for processing the audio input received from the remote operator, one of
i) a first audio interface (210) of the local computing device (200), the first audio interface (210) being configured for processing audio input, wherein a first audio device (400) is connected to the first audio interface (210) and wherein the first audio device (400) is configured to output the audio input processed by the first audio interface (210) to a local operator of a medical imaging device (500), and
ii) a second audio interface (220) of the local computing device (200), the second audio interface (220) being configured for processing audio input, wherein a second audio device (510) is connected to the second audio interface (220) and wherein the second audio device (510) is configured to output the audio input processed by the second audio interface (220) to a patient situated in the medical imaging device (500);
outputting (630) the audio input processed by the first audio interface (210) via the first audio device (400) when the first audio interface (210) is selected based on the received control input; and
outputting (630) the audio input processed by the second audio interface (220) via the second audio device (510) when the second audio interface (220) is selected based on the received control input.

9. The method (600) of claim 8, wherein the selecting step comprises:
selecting the first audio interface (210) per default when no control input from the remote operator is received and selecting the second audio interface (220) when the control input is received from the remote operator.

10. The method (600) of claim 9, wherein
the control input is received from the remote operator for a period of time, the method (600) further comprising:
changing the selection from the second audio interface (220) to the first audio interface (210) after the period of time lapses.

11. The method (600) of any of claims 8 to 10, wherein the first and second audio interfaces (210, 220) are configurable to be assigned to either the first audio device (400) or the second audio device (510), the method (600) further comprising:
receiving configuration input from the local operator indicating that the first audio interface (210) is to be assigned to the first audio device (400) and that the second audio interface (220) is to be assigned to the second audio device (510).

12. The method (600) of any of claims 8 to 11 wherein
the audio input processed by the first audio interface (210) for output to the local operator is part of an audio/video call held between the local operator and the remote operator, and wherein the audio input processed by the second audio interface (220) for output to the patient situated in the medical imaging device (500) is for communication between the remote operator and the patient.

13. The method (600) of any of claims 8 to 12, wherein
the control input is indicative of the remote operator actuating an input element; and, optionally, wherein
the input element is a button.

14. The method (600) of claim 13, wherein the local computing device (200) further comprises a display; and the method (600) further comprises:
triggering, via the display, an indication to the local operator indicative of the remote operator actuating the input element.

15. A computer program product (700) comprising instructions (710) that, when executed on at least one processor (230), cause the at least one processor (230) to carry out the method (600) according to any one of claims 8 to 14.
